# EUROPEAN PATENT SPECIFICATION

(11) **EP 4 083 186 B1**
(45) Date of publication and mention of the grant of the patent: **21.05.2025**
(21) Application number: 20905080.6
(22) Date of filing: 25.12.2020
(51) Int. Cl.: C12N 1/00, C12N 1/04, C12N 5/071, C12N 5/0775, C12N 5/077

(54) **CELL GROWTH REGULATOR**
ZELLWACHSTUMSREGULATOR
RÉGULATEUR DE CROISSANCE CELLULAIRE

(30) Priority: 27.12.2019 JP 2019239572
(43) Date of publication of application: 02.11.2022
(73) Proprietor: Bourbon Corporation, Kashiwazaki-shi, Niigata 945-8611 (JP)
(72) Inventor: TAKIZAWA Sakiko, Kashiwazaki-shi, Niigata 945-8611 (JP); FUJIMOTO Shunsuke, Kashiwazaki-shi, Niigata 945-8611 (JP); TAKASAKA Mieko, Matsumoto-shi, Nagano 390-8621 (JP); SASAKI Katsunori, Yokohama-shi, Kanagawa 236-0012 (JP)
(74) Representative: Elkington and Fife LLP
(86) International application number: PCT/JP2020/048636
(87) International publication number: WO 2021/132539

(56) References cited:
- EP-A1- 1 577 396
- WO-A1-2014/077289
- WO-A1-2015/194643
- M. GHADIRI ET AL: "Layered silicate clay functionalized with amino acids: wound healing application", RSC ADV., vol. 4, no. 67, 1 January 2014 (2014-01-01), pages 35332 - 35343, XP055753025, DOI: 10.1039/C4RA05216A
- SAVASKAN N E ET AL: "Dissection of mitogenic and neurodegenerative actions of cystine and glutamate in malignant gliomas", ONCOGENE, NATURE PUBLISHING GROUP UK, LONDON, vol. 30, no. 1, 30 August 2010 (2010-08-30), pages 43 - 53, XP037742068, ISSN: 0950-9232, [retrieved on 20100830], DOI: 10.1038/ONC.2010.391
- CHALISOVA N I ET AL: "Effect of amino acids on cell proliferation and protein p53 expression in organotypic culture of lymphoid tissue from neonatal rats", CELL BIOLOGY INTERNATIONAL, ACADEMIC PRESS, GB, vol. 32, no. 12, 1 December 2008 (2008-12-01), pages 1574 - 1577, XP025672250, ISSN: 1065-6995, [retrieved on 20081114], DOI: 10.1016/J.CELLBI.2008.09.005
- LOMBARDI G ET AL: "Glutamate modulation of human lymphocyte growth: in vitro studies", BIOCHEMICAL AND BIOPHYSICAL RESEARCH COMMUNICATIONS, ELSEVIER, AMSTERDAM NL, vol. 318, no. 2, 28 May 2004 (2004-05-28), pages 496 - 502, XP004505408, ISSN: 0006-291X, DOI: 10.1016/J.BBRC.2004.04.053
- DATABASE COMPENDEX [online] ENGINEERING INFORMATION, INC., NEW YORK, NY, US; 2014, GHADIRI M ET AL: "Layered silicate clay functionalized with amino acids: Wound healing application", Database accession no. E20143518118995
- GHADIRI M ET AL: "Layered silicate clay functionalized with amino acids: Wound healing application", RSC ADVANCES 2014 ROYAL SOCIETY OF CHEMISTRY GBR, vol. 4, no. 67, 2014, pages 35332 - 35343, DOI: 10.1039/C4RA05216A

## Description

### TECHNICAL FIELD

The present disclosure relates to a cell proliferation inhibitor comprising glutamic acid and xylose, and a method for regulating the proliferation of cells using the same.

### BACKGROUND ART

In biology, cultured cells artificially cultured *ex vivo* are widely used in *in vitro* experimental systems, regenerative medicine and cell therapy. In order to keep the properties of the cells constant, it is necessary to continue to proliferate the cells in a medium by subculture. However, when non-established cells that are limited in number of passages (life span) such as primary cultured cells and finite cell lines are subcultured, their properties are often changed.

Therefore, when the properties of cultured cells are kept constant for a medium to long period, the cells are commonly cryopreserved. However, cells that are vulnerable to freezing and thawing, such as primate cells, are significantly damaged by cryopreservation.

Further, in the cases of cells having a high proliferation speed and cultured cells having a low proliferation speed, the adjustment of the cell concentration to a desired value, at the time of using the cells, is difficult so that skill is required. A method of inhibiting cell proliferation by temporarily changing the environmental temperature of cells to a low temperature such as 4°C is also known, but the time capable of inhibiting is only for several hours.

Furthermore, among cultured cells, stem cells are known to lose their undifferentiated property (differentiation potency) which is one of the properties of the cells, upon subculture. Therefore, in the case of mouse ES cells, it is necessary to add, for example, LIF (leukemia inhibitory factor) in order to maintain the undifferentiated property. Further, in the case of primate ES cells, it is necessary to add an undifferentiation maintenance factor or to perform the culture in coexistence of feeder cells (see JP 2007-228815 A (Patent Document 1)). However, many of these undifferentiation maintenance factors are expensive, and performing the culture in coexistence of feeder cells is complicated.

Therefore, there is demanded a method for simply regulating the proliferation of a cell while maintaining the properties of the cell, without need to subculture the cell, by a means other than cryopreservation.

As the means other than cryopreservation, for example, JP 2008-104407 A (Patent Document 2) discloses a method for preserving an adherent cell characterized in that the adherent cell is preserved under refrigerated conditions using a cell preservation liquid containing a reducing sugar. This method, however, necessarily requires refrigeration at a low temperature, and the types of cells capable of preserving are limited.

Xylose is one of the constituent sugars of sugar chains and plays an important role in the living body, such as communication between cells. In addition, xylose is known to be abundantly contained in woody biomass, and it is required to expand its use as an unused resource in various fields.

The present disclosers have previously confirmed the effects of various saccharides (glucose, xylose, galactose, etc.) on mouse ES cells, and reported the possibility that xylose can maintain undifferentiation of the mouse ES cells (Tadayuki YOKOYAMA et al., "Proliferation of mouse ES cells in environments containing various sugars and effect on EB formation," Proceedings of The 7th Congress of Japanese Society for Regenerative Medicine, Vol. 7, p. 239, 2008 (Non-Patent Document 1) and Tadayuki YOKOYAMA et al., "Reviews on effectiveness in maintaining undifferentiation of mouse ES cells in xylose-containing environments," Proceedings of The 8th Congress of Japanese Society for Regenerative Medicine, Vol. 8, p. 221, 2009 (Non-Patent Document 2)).

Also, the present disclosers have reported that, despite the previous thought that pluripotent stem cells, when cultured in a medium containing xylose in place of glucose contained in a common medium, cannot utilize saccharides other than glucose as energy and thus cannot survive, it is possible to maintain the survival of pluripotent stem cells and inhibit the proliferation thereof at a normal culture temperature for a long period exceeding one week (Patent Document 3).

However, it is still an important subject to provide a method for simply regulating the proliferation of a wide variety of primate cells such as fibroblasts and mesenchymal stem cells while maintaining their properties.

### Prior Art Documents

### Patent Documents

Patent Document 1: JP 2007-228815 A
Patent Document 2: JP 2008-104407 A
Patent Document 3: JP 6042099 B

### Non-Patent Documents

Non-Patent Document 1: Tadayuki YOKOYAMA et al., "Proliferation of mouse ES cells in environments containing various sugars and effect on EB formation," Proceedings of The 7th Congress of Japanese Society for Regenerative Medicine, Vol. 7, p. 239, 2008

Non-Patent Document 2: Tadayuki YOKOYAMA et al., "Reviews on effectiveness in maintaining undifferentiation of mouse ES cells in xylose-containing environments," Proceedings of The 8th Congress of Japanese Society for Regenerative Medicine, Vol. 8, p. 221, 2009
Savaskan et al. (Oncogene, 2011, vol.30, 43-53) report the effect of cystine and glutamate in malignant gliomas. Ghadiri et al. (RSC Adv., 2014, vol.4, 35332-35343) reports the use of layered silicate clay functionalized with amino acids, such as glutamic acid, for wound healing application.

### SUMMARY OF THE INVENTION

The present disclosers have now found that, when a specific amino acid is used as a cell proliferation inhibitor, it is possible to simply regulate the proliferation of a wide variety of primate cells such as fibroblasts and mesenchymal stem cells while maintaining their properties. The present disclosure is based on such knowledge.

The present invention relates to the use of glutamic acid and xylose for inhibiting the proliferation of a cell in a cell culture medium, wherein the cell is selected from a human fibroblast and a human mesenchymal stem cell.

The present invention further relates to a cell culture medium comprising 0.5 µM or more of glutamic acid, xylose, and a cell which is maintained in the medium, wherein the cell is selected from a human fibroblast and a human mesenchymal stem cell.

The invention further relates to a method for regulating the proliferation of cells, comprising maintaining the cell by using a cell culture medium comprising 0.5 µM or more of glutamic acid, xylose, and a cell which is maintained in the medium, wherein the cell is selected from a human fibroblast and a human mesenchymal stem cell.

The cell proliferation inhibitor of the present disclosure can be used to inhibit the proliferation of the cell while maintaining the properties of the cell under normal culturing conditions. Also, the cell proliferation inhibitor of the present disclosure can be advantageously utilized to inhibit the proliferation of the cells while inhibiting the killing of the cells. Therefore, the cell proliferation inhibitor of the present disclosure is extremely useful in preservation and maintenance of the cells for a short period, without need for cryopreserved.

In addition, the method for regulating the proliferation of cells according to the present disclosure is very simple without requiring any skill, since only an operation of replacing the normal medium with the medium comprising the cell proliferation inhibitor of the present disclosure is performed.

Furthermore, in the case where the cells is pluripotent stem cells, when a medium comprising the cell proliferation inhibitor of the present disclosure is used, it is possible to maintain the pluripotent stem cell while inhibiting the proliferation of the cell and to retain the undifferentiated property and totipotency of the cell without adding an undifferentiation maintenance factor or culturing the cell in coexistence of a feeder cell. Therefore, it is possible to maintain even a pluripotent stem cell that is vulnerable to freezing for a short period while retaining its undifferentiated property and totipotency as is the case with cryopreservation.

### BRIEF DESCRIPTION OF THE DRAWINGS

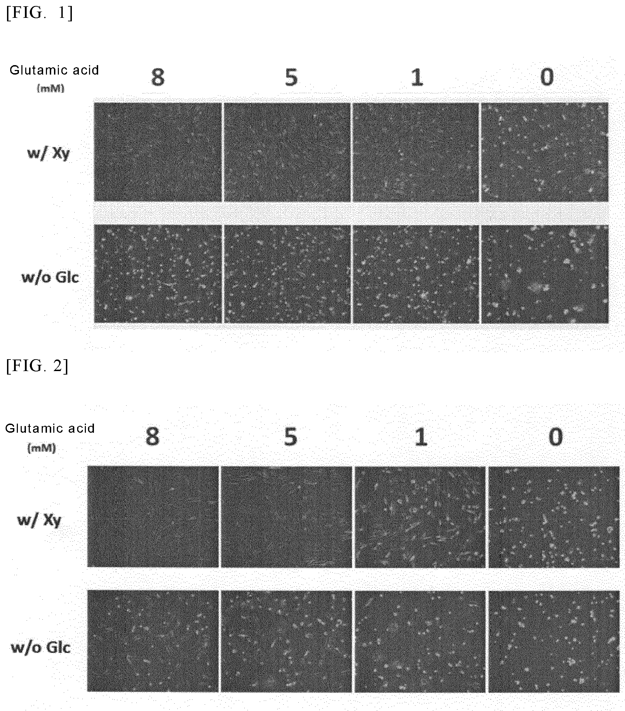

Fig. 1 shows results of microscopic observation when human fibroblasts were cultured in cell maintenance media (xylose DMEM basal medium and glucose-free DMEM basal medium) in which the addition amount of glutamic acid was adjusted (phase-contrast images on Day 9 of culture, magnification: 100 times).
Fig. 2 shows results of microscopic observation when human mesenchymal stem cells were cultured in cell maintenance media (xylose DMEM basal medium and glucose-free DMEM basal medium) in which the addition amount of glutamic acid was adjusted (phase-contrast images on Day 9 of culture, magnification: 100 times).

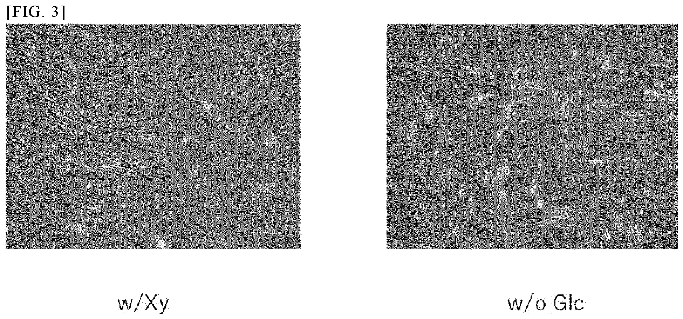

Fig. 3 shows results of microscopic observation when human mesenchymal stem cells were cultured in cell maintenance media (xylose DMEM basal medium and glucose-free DMEM basal medium) in which the 8mM of glutamic acid was added to adjust (phase-contrast images on Day 12 of culture, magnification: 100 times).

### DETAILED DESCRIPTION OF THE INVENTION

### Cell proliferation inhibitor

The cell proliferation inhibitor according to the present disclosure comprises glutamic acid, as described above.

The cell proliferation inhibitor comprises glutamic acid as an active ingredient. The "active ingredient," as used herein, means a component required to provide the cell proliferation inhibitory effect, which is an object of the present disclosure. The cell proliferation inhibitory effect means maintaining the survival of cells and inhibiting the proliferation of the cells. The cells can be preserved by inhibiting the proliferation of the cells.

The cell proliferation inhibitor is used together with xylose, as described above.

Xylose is a pentose monosaccharide, which is abundantly contained in woody biomass and is referred to also as wood sugar. The xylose used in the present disclosure may be naturally abundant D-xylose, or synthetically prepared L-xylose or DL-xylose. The xylose used in the present disclosure is preferably D-xylose.

As used herein, the cells are human fibroblasts and human mesenchymal stem cells. The cell used in the present disclosure is preferably a fibroblast or a mesenchymal stem cell, since the cells has an extremely high proliferation property and it is necessary to retain its traits such as undifferentiated property.

The fibroblast is one of cells constituting connective tissue and includes cells that produce dermal components such as collagen, elastin and hyaluronic acid.

The mesenchymal stem cell is a somatic stem cell derived from mesodermal tissue (mesenchyme). The mesenchymal stem cell is a cell capable of exerting the ability to differentiate into cells belonging to the mesenchymal system, and is expected to be applied to regenerative medicine such as reconstruction of bone, blood vessels and cardiac muscles. The mesenchymal stem cell can be classified depending on the tissue from which it is collected, and examples thereof include bone marrow-derived stem cells and adipose tissue-derived stem cells.

The primary cultured cells used in the present disclosure means cells obtained by first seeding and culturing tissues or cells collected from a living body. The finite cell line used in the present disclosure means subcultured cells which is limited in number of passages (life span). The proliferation inhibitor of the present disclosure can be used to maintain and preserve the primary cultured cells and the finite cell lines whose properties easily change.

The cell proliferation inhibitor of the present disclosure is expected to be used in substance responsiveness tests and clinical tests in a state where biological activity is stopped.

According to one aspect of the present disclosure, there is provided a cell culture medium comprising the cell proliferation inhibitors of the present disclosure, and a cell which is maintained in the medium, wherein the cell is selected from a human fibroblast and a human mesenchymal stem cell.

The "cell culture medium" means a culture medium suitable for culturing the cells described above or a composition thereof, and enables a constant number of cells to survive for a constant period, while retaining the properties of the cells without causing any serious damage that affects the survival of the cells. The cell culture medium may be in the form of a powder or a liquid.

The content of glutamic acid in the cell culture medium can be appropriately changed depending, for example, on the type of cells to be cultured, the purpose of culture, and the type of basal medium. The content of glutamic acid in the basal medium is set to 0.5 µM or more, for example, 0.5 µM to 10 mM, more preferably 0.5 mM to 10 mM, further preferably 1 to 10 mM, still further preferably 5 mM to 8 mM, even still further preferably 8 mM.

Examples of the basal medium include Dulbecco's Modified Eagle's Medium (DMEM), Eagle's Minimum Essential Medium (MEM), α-Modified Eagle's Minimum Essential Medium (α-MEM), Ham's F12 medium, MCDB medium, Fischer's medium and RPMI-1640 medium, in which glucose is replaced with xylose. DMEM is preferred.

The content of xylose in the cell culture medium is not particularly limited, and can be appropriately changed depending, for example, on the type of cells to be cultured, the purpose of culture, and the type of basal medium. The content of xylose in the medium may be equal to the amount of glucose in a normal basal medium, and can be set to, for example, 0.1 to 10.0 g/L, but is preferably 0.5 to 10.0 g/L, more preferably 0.5 to 5.0 g/L, further preferably 0.8 to 5.0 g/L, still further preferably 0.8 to 3.5 g/L. The present disclosure can exhibit a sufficient effect even in the case of a low content of xylose in the medium according to the present disclosure, but xylose is not toxic and has excellent water solubility, and thus, normally, substantially does not cause any problem even if added in a large amount.

Further, the cell culture medium is preferably used without addition of glucose, from the viewpoint of inhibiting the cell proliferation due to glucose. Thus, according to one aspect, the cell culture medium is substantially free of glucose.

When xylose is introduced into the medium, xylose is preferably introduced by replacing glucose in a medium used in conventional cell culture with xylose.

Serum or a serum substitute or any other component may be added to the medium, as needed. Any known serum or serum substitute can be used. Examples of the serum include FBS and FCS, and examples of the serum substitute include KSR.

Examples of the other component include non-essential amino acids and pH adjusters.

The serum or serum substitute or other component to be added to the medium is preferably one from which a saccharide has been removed by membrane treatment. The membrane treatment can be performed, for example, by using a dialysis membrane 36/32 (manufactured by EIDIA Co., Ltd.).

In addition, the cell culture medium is preferably used as a cell maintenance medium. The "cell maintenance medium" means a culture medium suitable for culturing the cells described above or a composition thereof, and enables a constant number of cells to survive for a constant period, while retaining the undifferentiated property and differentiation pluripotency of undifferentiated cells and the traits of fibroblasts such as collagen production ability.

The cell maintenance medium comprises the cell proliferation inhibitor described above, and thus can retain the properties of cells even though it is substantially free of an undifferentiation maintenance factor other than glutamic acid and xylose, which is considered to be an essential component of cell media. Thus, according to one aspect of the present disclosure, the medium of the present disclosure comprises the cell proliferation inhibitor and is substantially free of an undifferentiation maintenance factor other than glutamic acid and xylose. Examples of the undifferentiated maintenance factor other than glutamic acid and xylose include bFGF (basic fibroblast growth factor), LIF and feeder cell-derived components.

Also, according to one aspect of the present disclosure, there is provided a method for regulating the proliferation of cells, comprising maintaining cells by using the cell culture medium according to the present disclosure.

The "regulating the proliferation of cells" means stopping the proliferation ability of cells at a desired time, maintaining the cell concentration without imparting to the cell any serious damage that affects the survival of the cells, and then restarting the proliferation of the cells at a desired time. Specifically, the cell proliferation can be regulated by replacing the cell maintenance medium of the present disclosure with a normal cell culture medium. Depending on the cells, the proliferation speed after the restart of proliferation can be increased to the same level as or a higher level than that in normal culture.

The proliferation of cells may be regulated at a normal cell culture temperature and under normal environmental conditions.

The period for inhibiting the proliferation of cells can be appropriately changed depending on the type of cells to be cultured, the purpose of culture, the type of basal medium, the culture temperature, and the like. For example, when DMEM in which glucose is replaced with xylose in the medium is used as the cell maintenance medium of the present disclosure and is cultured under normal culture conditions, the cell proliferation can be expected to be inhibited for at least one month, preferably for at least 26 days, more preferably for at least 21 days, further preferably for at least 14 days, still further preferably for at least 8 days.

The cell maintenance medium may be exchanged during the proliferation inhibiting period. The medium exchange provides the advantage of retaining the property of cells even during long-term culture because of the amino acid, protein components and the like in the medium can be replenished.

### EXAMPLES

Hereinafter, the present disclosure will be described in detail by way of examples.

Unless otherwise specified, the units and measurement methods of the present disclosure conform to the provisions of the Japanese Industrial Standards (JIS). Unless otherwise specified, the "parts" and "%" mean "parts by mass" and "% by mass", respectively, and the temperature is expressed in degrees centigrade.

### Glutamic acid addition test

### (1) Preparation of medium

Basal media for cell culture were prepared so as to have the following compositions.

### DMEM basal medium (hereinafter referred to also as "w/Glc")

### (Glucose content: 1.0 g/L, xylose content: 0 g/L)

In Dulbecco's Modified Eagle's Medium (DMEM; Cell Science & Technology Institute, Inc.), the amounts of components to be added were adjusted to attain 10% by volume of dialysis FBS (GE Healthcare), 50 U/mL of penicillin, and 50 µg/mL of streptomycin (FUJIFILM Wako Pure Chemical Corporation).

### Cell maintenance medium

### (i) Xylose DMEM basal medium (hereinafter referred to also as "w/Xy")

### (Glucose content: 0 g/L, xylose content: 1.0 g/L)

In modified DMEM (M-DMEM, obtained from Cell Science & Technology Institute, Inc.) in which glucose in the DMEM basal medium had been replaced with D-xylose, the amounts of components to be added were adjusted to attain 10% by volume of dialysis FBS, 50 U/mL of penicillin, and 50 µg/mL of streptomycin (FUJIFILM Wako Pure Chemical Corporation) to prepare w/Xy.

### (ii) Glucose-free DMEM basal medium (hereinafter referred to also as "w/o Glc")

### (Glucose content: 0 g/L, xylose content: 0 g/L)

In modified DMEM (M-DMEM, obtained from Cell Science & Technology Institute, Inc.) in which glucose in the DMEM basal medium had been removed, the amounts of components to be added were adjusted to attain 10% by volume of dialysis FBS, 50 U/mL of penicillin, and 50 µg/mL of streptomycin (FUJIFILM Wako Pure Chemical Corporation) to prepare w/o Glc.

### (2) Cell

### Preparation of human fibroblast

Human fibroblasts (Catalog No. CC-2511) were cultured in a gelatin-coated cell culture dish (100-mm culture dish, obtained from SANPLATEC CO., LTD.) at 37°C in the presence of 5% CO₂, and used. As the medium, the DMEM basal medium was used.

### Preparation of human mesenchymal stem cell

Human mesenchymal stem cells (manufactured by Lonza, Catalog No. PT-5006) were cultured in a cell culture dish (100-mm culture dish, obtained from SANPLATEC CO., LTD.) coated with 10 µg/ml Fibronectin (Promocell) at 37°C in the presence of 5% CO₂, and used. As the medium, the DMEM basal medium was used.

### (3) Test for confirming cell proliferation inhibitory effect

### Example 1

After cell culture, the cells were recovered using a 0.1% by weight trypsin/1 mM EDTA solution, and seeded on a 12-well plate (obtained from Corning Co., Ltd.), and cultured overnight at 37°C in the presence of 5% CO₂ using the above DMEM basal medium.

The DMEM basal medium was replaced with the desired cell maintenance medium, and the cells were cultured ("Day 0 after culture"). No medium exchange was performed during the culture period.

Further, the same culture test as described above was conducted except that 1 mM, 5 mM or 8 mM of glutamic acid was added to the basal medium for cell culture.

### Observation of cell state

The cells on Day 9 after culture were observed with a phase-contrast microscope (IX71, manufactured by Olympus Corporation, magnification: 100 times).

The results when human fibroblasts were used were as shown in Fig. 1.

In the xylose DMEM basal medium (w/Xy), it was observed that the number of dead cells decreased and cell proliferation was inhibited as the glutamic acid concentration increased. In particular, when the glutamic acid concentration was in the range of 1 to 8 mM, dead cells were hardly observed.

Also in the glucose-free DMEM basal medium (w/o Glc), it was observed that the number of dead cells decreased and cell proliferation was inhibited as the glutamic acid concentration increased.

The results when human mesenchymal stem cells were used were as shown in Fig. 2, and the same tendency as that when human fibroblasts were used was observed.

In the xylose DMEM basal medium (w/Xy), it was observed that the number of dead cells decreased and cell proliferation was inhibited as the glutamic acid concentration increased. In particular, when the glutamic acid concentration was in the range of 5 to 8 mM, dead cells were hardly observed.

Also in the glucose-free DMEM basal medium (w/o Glc), it was observed that the number of dead cells decreased and cell proliferation was inhibited as the glutamic acid concentration increased.

It was observed that the cell state was better and the number of dead cells was significantly small in w/Xy compared with in w/o Glc.

As a control test, the DMEM basal medium was used instead of the cell maintenance medium to conduct the test for confirming the cell proliferation inhibitory effect in the same procedures as described above. As a result, cell proliferation was confirmed while cell death was hardly confirmed, in either case where human fibroblasts or human mesenchymal stem cells were used.

### Example 2

The same culture test as in Example 1 was conducted except that the medium was exchanged every three days during the culture period.

Tables 1 to 2 indicate the number of cells for each culture day. Table 1 shows the results of using the human fibroblasts, and Table 2 shows the results of using the human mesenchymal stem cells. Note that the number of cells is an average of the numbers of cell counts in three wells, and the counts is measured by the following method.

After removing the culture supernatant of the cells by suction and washing the cells by using PBS, the cells were peeled off by using 0.1% trypsin/1 mM EDTA solution (FUJIFILM Wako Pure Chemical Corporation). The reaction of the releasing solution was stopped by adding the medium, and the cells were recovered. And then, the cell counts was conducted by using the Countess^{™}II Automated Cell Counter(Thermo Fisher Scientific).

**[Table 1]**

| Human fibroblast | Number of cells (×10⁵ cells/cm²) | | | |
|---|---|---|---|---|
| Culture days | Day 0 | Day 6 | Day 12 | Day 18 |
| Medium: w/Glc | 0.07 | 0.65 | 1.39 | 1.57 |
| Medium: w/Glc | 0.07 | 0.56 | 1.46 | 1.72 |
| Glutamic acid: 8mM | | | | |
| Medium: w/Xy | 0.07 | 0.37 | 1.13 | 1.28 |
| Glutamic acid: 8mM | | | | |
| Medium: w/o Glc | 0.07 | 0.27 | 0.08 | - |
| Glutamic acid: 8mM | | | | |

From Table 1, in the human fibroblasts, it was possible to confirm that the cell proliferation was inhibited till 6 days after by adding the Glutamic acid compared with the control test with w/Glc medium. Moreover, when w/Xy medium instead of w/Glc medium was used, the cell proliferation inhibitory effect was remarkable, and it was possible to inhibit the cell proliferation for a longer period. On the other hand, when w/o Glc medium instead of w/Glc medium was used, the number of cells after 12 days was remarkably less, and the dead cells occurred, unlike the w/Xy medium.

**[Table 2]**

| Human mesenchymal stem cell | Number of cells (×10⁴ cells/cm²) | | | | | | |
|---|---|---|---|---|---|---|---|
| Culture days | Day 0 | Day 3 | Day 6 | Day 9 | Day 12 | Day 15 | Day 18 |
| Medium: w/Glc | 0.356 | 1.16 | 3.32 | 3.26 | 3.78 | 5.53 | 6.23 |
| Medium: w/Glc | 0.356 | 1.12 | 2.96 | 3.23 | 3.75 | 4.46 | 5.37 |
| Glutamic acid: 8mM | | | | | | | |
| Medium: w/Xy | 0.356 | 0.855 | 0.998 | 1.65 | 1.87 | 2.77 | 3.90 |
| Glutamic acid: 8mM | | | | | | | |
| Medium: w/o Glc | 0.356 | 0.591 | 0.662 | 1.06 | 1.04 | 1.50 | 1.58 |
| Glutamic acid: 8mM | | | | | | | |

From Table2, in the human mesenchymal stem cells, it was possible to inhibit the cell proliferation till 18 days after by adding the Glutamic acid compared with the control test with w/Glc medium. Moreover, same as in the human fibroblasts, when w/Xy medium instead of w/Glc medium was used, the cell proliferation inhibitory effect was remarkable. Further, when w/o Glc medium instead of w/Glc medium was used, as shown in Figure 3, the ratio of whitening around cells ware large, and the conditions of cells deteriorated, unlike the w/Xy medium.

The cell proliferation inhibitor of the present disclosure comprising glutamic acid can inhibit the proliferation of the cells. The cell proliferation inhibitor of the present disclosure can remarkably inhibit the proliferation of the cells and inhibit the proliferation of the cells for a longer period by using together with xylose.

Accordingly, the cell proliferation inhibitor of the present disclosure is extremely useful in preservation and maintenance of the cells for a short period, without need for cryopreserved.

## Claims

1. Use of glutamic acid and xylose for inhibiting the proliferation of a cell in a cell culture medium, wherein the cell is selected from a human fibroblast and a human mesenchymal stem cell.

2. A cell culture medium, comprising 0.5 µM or more of glutamic acid, xylose, and a cell which is maintained in the medium, wherein the cell is selected from a human fibroblast and a human mesenchymal stem cell.

3. The cell culture medium according to claim 2, wherein the medium comprises 0.1 to 10.0 g/L of xylose.

4. A method for regulating the proliferation of cells, comprising maintaining a cell by using a cell culture medium, comprising 0.5 µM or more of glutamic acid, xylose, and the cell which is maintained in the medium, wherein the cell is selected from a human fibroblast and a human mesenchymal stem cell.

5. The method according to claim 4, wherein the cell culture medium comprises 0.1 to 10.0 g/L of xylose.

## Patentansprüche

1. Verwendung von Glutaminsäure und Xylose zur Hemmung der Proliferation einer Zelle in einem Zellkulturmedium, wobei die Zelle aus einem humanen Fibroblasten und einer humanen mesenchymalen Stammzelle ausgewählt ist.

2. Zellkulturmedium, umfassend 0,5 µM oder mehr Glutaminsäure, Xylose und eine in dem Medium gehaltene Zelle, wobei die Zelle aus einem humanen Fibroblasten und einer humanen mesenchymalen Stammzelle ausgewählt ist.

3. Zellkulturmedium nach Anspruch 2, wobei das Medium 0,1 bis 10,0 g/l Xylose umfasst.

4. Verfahren zum Regulieren der Proliferation von Zellen, umfassend das Halten einer Zelle unter Verwendung eines Zellkulturmediums, umfassend 0,5 µM oder mehr Glutaminsäure, Xylose und die Zelle, die in dem Medium gehalten wird, wobei die Zelle ausgewählt ist aus einem humanen Fibroblasten und einer humanen mesenchymalen Stammzelle.

5. Verfahren nach Anspruch 4, wobei das Zellkulturmedium 0,1 bis 10,0 g/l Xylose umfasst.

## Revendications

1. Utilisation d'acide glutamique et de xylose pour inhiber la prolifération d'une cellule dans un milieu de culture cellulaire, dans laquelle la cellule est choisie parmi un fibroblaste humain et une cellule souche mésenchymateuse humaine.

2. Milieu de culture cellulaire, comprenant 0,5 µM ou plus d'acide glutamique, de xylose et une cellule qui est maintenue dans le milieu, dans lequel la cellule est choisie parmi un fibroblaste humain et une cellule souche mésenchymateuse humaine.

3. Milieu de culture cellulaire selon la revendication 2, dans lequel le milieu comprend 0,1 à 10,0 g/L de xylose.

4. Procédé de régulation de la prolifération de cellules, comprenant le maintien d'une cellule en utilisant un milieu de culture cellulaire, comprenant 0,5 µM ou plus d'acide glutamique, de xylose et la cellule qui est maintenue dans le milieu, dans lequel la cellule est choisie parmi un fibroblaste humain et une cellule souche mésenchymateuse humaine.

5. Procédé selon la revendication 4, dans lequel le milieu de culture cellule comprend 0,1 à 10,0 g/L de xylose.
